(19) Europäisches Patentamt
European Patent Office
Office européen des brevets

(11) Veröffentlichungsnummer: **0 261 552**
**A1**

(12) **EUROPÄISCHE PATENTANMELDUNG**

(21) Anmeldenummer: **87113445.8**

(22) Anmeldetag: **15.09.87**

(51) Int. Cl.⁴: **C12N 15/00** , C07H 21/04 , C07K 7/36 , C12N 1/20 , //C12P21/02,C12R1:19

(30) Priorität: **20.09.86 DE 3632037**

(43) Veröffentlichungstag der Anmeldung:
**30.03.88 Patentblatt 88/13**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hein, Friedrich, Dr.**
**Erlesring 40**
**D-6234 Hattersheim am Main(DE)**
Erfinder: **Jansen, Hans Willi, Dr.**
**Drosselweg 1**
**D-6230 Frankfurt am Main 80(DE)**
Erfinder: **Müller, Hubert, Dr.**
**Berliner Ring 20**
**D-6233 Kelkheim (Taunus)(DE)**
Erfinder: **Uhlmann, Eugen, Dr.**
**Zum Talblick 31**
**D-6246 Glashütten/Taunus(DE)**

(54) **Gentechnisches Verfahren zur Herstellung von Salm-Calcitonin sowie Mittel zur Durchführung dieses Verfahrens.**

(57) Mit Hilfe einer speziellen DNA-Sequenz kann in einem gentechnischen Verfahren Salm-Calcitonin I mit zusätzlichem C-terminalem Glycin gewonnen werden. Das Gen wird vorteilhaft aus zwei Oligonukleotiden synthetisiert, in ein Hybridplasmid eingebaut und dieses in einem Wirtsorganismus zur Expression gebracht. Salm-Calcitonin I-Gly$^{33}$ ist enzymatisch in Salm-Calcitonin I umwandelbar.

EP 0 261 552 A1

Xerox Copy Centre

# Gentechnisches Verfahren zur Herstellung von Salm-Calcitonin sowie Mittel zur Durchführung dieses Verfahrens

Calcitonin ist als hypokalzämisches und hypophosphatämisches Peptidhormon an der Regulation des Serum-Kalziumspiegels beteiligt. Gemeinsame Strukturmerkmale von Calcitoninen aus verschiedenen Spezies sind eine Länge von 32 Aminosäuren, eine Disulfidbrücke zwischen den Cysteinresten 1 und 7 und eine C-terminale Prolinamidfunktion. Von den kommerziell erhältlichen Calcitoninen (Mensch, Schwein, Aal, Salm) ist Salm-Calcitonin das wirksamste. Calcitonin wird zur Behandlung von Störungen des Kalziumstoffwechsels eingesetzt. Dazu gehören Hyperkalzämie, Morbus Paget, verschiedene Formen der Osteoporose sowie das Sudeck-Syndrom. Die analgetische Wirkung des Calcitonins stellt einen weiteren aktuellen Aspekt dieses Hormons dar. Ferner ist Calcitonin bei der akuten Pankreatitis therapeutisch wirksam, indem es die Pankreasenzym-Sekretion hemmt und so die Normalisierung zahlreicher Krankheitsparameter beschleunigt.

Die gentechnische Herstellung von Polypeptiden mit einem Carbonsäureamid-Carboxyterminus wie Calcitonin über das entsprechende Peptid mit einem Glycyl-C-Terminus und dessen enzymatische Umwandlung ist bereits bekannt (Europäische Patentanmeldung mit der Veröffentlichungsnummer - im folgenden "EP-A" - 0 133 282).

In der EP-A 191 869 ist ein Verfahren zur Herstellung von Salm-Calcitonin I mit zusätzlichem N-terminalen Methionin, C-terminalen Glycin (Salm-Calcitonin I-$Gly^{33}$) und von Salm-Calcitonin I-Carboxamid beschrieben, wobei man in ein Expressionsplasmid ein Gen einbaut, dieses exprimiert und gegebenenfalls das Glycyl-Peptid enzymatisch in das Carboxamid umwandelt.

Die vorliegende Erfindung ist in den Patentansprüchen definiert. Sie betrifft insbesondere ein spezielles Gen zur Herstellung von Salm-Calcitonin I-$Gly^{33}$.

Der erste Schritt des gentechnologischen Verfahrens ist die chemische Synthese des speziellen Gens. Dabei werden der codierende Strang sowie der nichtcodierende Strang jeweils chemisch durchsynthetisiert und somit auf die enzymatische Ligationsreaktion, wie sie gemäß EP-A 0 191 869 und auch sonst normalerweise zum Aufbau eines Gens aus sich überlappenden Fragmenten verwendet wird (vgl. EP-A 0 133 282, 0 136 472, 0 155 590, 0 161 504, 0 163 249, 0 171 024, 0 173 149 oder 0 177 827), bewußt verzichtet.

Der genetische Code ist bekanntlich "entartet", d.h. daß nur für zwei Aminosäuren eine einzige Nukleotidsequenz codiert, während den restlichen 18 genetisch codierbaren Aminosäuren zwei bis sechs Tripletts zuzuordnen sind. Für die Synthese des Gens steht somit eine große Vielfalt von Codon-Kombinationen zur Auswahl. Es wurde nun gefunden, daß die DNA-Sequenz I (Anhang, dort wiedergegeben mit ATG-Startcodon, zwei Translations-Stopcodons und überhängenden Enden für unterschiedliche Restriktionsenzyme) besonders vorteilhaft ist.

Am 5'-Ende des codierenden Stranges befindet sich eine überhängende DNA-Sequenz entsprechend der Restriktionsendonuklease SphI, am 3'-Ende des codierenden Strangs dagegen die einzelsträngige überhängende Sequenz entsprechend dem Restriktionsenzym EcoRI. Diese beiden unterschiedlichen Erkennungssequenzen gewährleisten die Inserierung der DNA in Plasmide in der gewünschten Orientierung.

Zwischen diesen Erkennungssequenzen und den Codons für die Aminosäurefolge befindet sich am 5'-Ende des codierenden Stranges das Codon für die Aminosäure Methionin (das in der DNA-Sequenz I mit 0 beziffert ist). Am Ende dieses Stranges folgt auf das für Prolin codierende Triplett das Codon 33 für Glycin, gefolgt von zwei Translationsterminations-Tripletts (Stop-Codons).

Innerhalb des Strukturgens wurden eine Reihe von singulären Erkennungssequenzen für Restriktionsendonukleasen eingebaut, die Zugang zu Teilsequenzen des Salm-Calcitonins I schaffen sowie die Durchführung von Mutationen erlauben:

| Restriktionsenzym | Schnitt nach Nukleotid Nr. (codierender Strang) |
|---|---|
| SalI | 20 |
| HindIII | 39 |
| PstI | 67 |
| HpaII (MspII) | 88 |
| KpnI | 100 |
| ScrFI | 103 |

Bei der DNA-Sequenz I wurde weiterhin berücksichtigt, daß bei denjenigen Aminosäuren, denen mehrere Codons zuzuordnen sind, diese nicht gleichwertig sind, sondern in der jeweiligen Wirtszelle wie E. coli unterschiedliche Präferenzen zeigen. Weiterhin wurden palindromische Sequenzen auf ein Mindestmaß reduziert. Die DNA-Sequenz I läßt sich aus zwei chemisch synthetisierten Oligonukleotiden mit Längen von 109 und 117 Nukleotiden aufbauen.

Das synthetische Gen wird nach Inserierung in das handelsübliche Plasmid pUC19 (Figur 1) in E. coli kloniert. Aus dem reisolierten Plasmid kann die DNA-Sequenz I durch Verdauung mit SphI und EcoRI wiedergenommen werden.

Nach Umklonieren des Gens in einen Expressionsvektor wie pWH1 (EP-A 0 133 282) wird Salm-Calcitonin I-$Gly^{33}$ in Form eines Fusionsproteins mit einem bakteriellen Protein wie $\beta$-Galaktosidase oder Partialsequenzen desselben exprimiert Solche Fusionsproteine können dann in bekannter Weise chemisch gespalten werden.

Das Methionin am Aminoterminus kann durch Bromcyanspaltung abgetrennt werden, so daß der unerwünschte Anteil des Bakterienproteins im Fusionsprotein mit entfernt wird.

Die enzymatische Umwandlung des Glycylrestes 33 in die Aminogruppe der Prolinamid-Funktion im Salm-Calcitonin I ist an sich bekannt (EP-A 0 191 869 und 0 133 282; A.F. Bradbury et al., Nature 298 (1982) 686-688).

Der Einbau des synthetischen Gens in das Plasmid erfolgt in an sich bekannter Weise. Hierzu kann auf das Lehrbuch von Maniatis (Molecular Cloning, Maniatis et al., Cold Spring Harbor, 1982) verwiesen werden. Die Transformation des so erhaltenen Hybridplasmids in geeignete Wirtsorganismen, vorteilhaft E. coli, ist ebenfalls an sich bekannt und in dem vorstehend genannten Lehrbuch eingehend beschrieben. Die Gewinnung des exprimierten Proteins und dessen Reinigung können nach den in der EP-A 0 191 869 und 0 133 282 genannten Verfahren erfolgen.

Die erfindungsgemäße DNA-Sequenz I, die damit erhaltenen Hybridplasmide und die transformierten Wirtsorganismen sind ebenfalls Gegenstand der Erfindung.

In den folgenden Beispielen werden einige Ausgestaltungen der Erfindung im einzelnen erläutert. Prozentangaben beziehen sich hierbei auf das Gewicht, wenn nichts anderes angegeben ist.

## Beispiele

### 1. Chemische Synthese eines einzelsträngigen Oligonukleotids

Am Beispiel des codierenden Stranges der DNA-Sequenz I wird die Synthese der beiden Genbausteine erläutert. Für die Festphasensynthese wird das am 3'-Ende stehende Nukleosid, im vorliegenden Fall also Guanosin (Nukleotid Nr. 113), über die 3'-Hydroxyfunktion kovalent an einen Träger gebunden verwendet. Trägermaterial ist mit langkettigen Aminoalkylresten funktionalisiertes CPG ("Controlled Pore Glass").

In den folgenden Syntheseschritten wird die Basenkomponente als 5'-O-Dimethoxytritylnukleosid-3'-phosphorigsäure-$\beta$-cyanoethylester-dialkylamid eingesetzt, wobei das Adenin als $N^6$-Benzoyl-Verbindung, das Cytosin als $N^4$-Benzoyl-Verbindung, das Guanin als $N^2$-Isobutyryl-Verbindung und das Thymin ohne Schutzgruppe vorliegen.

25 mg des polymeren Trägers, der 0,2 $\mu$mol $N^2$-Isobutyrylguanosin gebunden enthält, werden nacheinander mit folgenden Agentien behandelt:

A) Acetonitril
B) 3 % Trichloressigsäure in Dichlormethan
C) Acetonitril
D) 5 µmol des entsprechenden Nukleosid-phosphits und 25 µmol Tetrazol in 0,15 ml wasserfreiem Acetonitril
E) Acetonitril
F) 20 % Acetanhydrid in Tetrahydrofuran mit 40 % Lutidin und 10 % Dimethylaminopyridin
G) Acetonitril
H) 3 % Jod in Lutidin/Wasser/Tetrahydrofuran im Volumenverhältnis 5:4:1.

Unter "Phosphit" wird hierbei der Desoxyribose-3'-monophosphorigsäure-mono-β-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden.

Nach abgeschlossener Synthese erfolgt die Abspaltung der Dimethoxytritylgruppe wie in A) bis C) beschrieben. Durch die Behandlung mit Ammoniak wird das Oligonukleotid vom Träger gespalten und zugleich werden die β-Cyanoethylgruppen eliminiert. Eine zwei-bis dreitägige Behandlung des Oligomeren mit konzentriertem Ammoniak spaltet die Aminoschutzgruppen der Basen quantitativ ab. Das so erhaltene Rohprodukt wird durch Polyacrylamid-Gelelektrophorese gereinigt.

2. Herstellung eines Hybridplasmids, das die DNA-Sequenz I enthält

Je 100 pmol des 109-mer Oligonukleotids (codierender Strang) und 117-mer Oligonukleotids (nichtcodierender Strang) werden zusammen in 50 µl 50 mM Tris-HCl-Puffer (pH 7,6), 10 mM Magnesiumchlorid und 10 mM Dithiothreitol gelöst, diese Lösung 5 Minuten auf 95°C erhitzt und binnen zwei Stunden auf Raumtemperatur abgekühlt. Der hybridisierte DNA-Doppelstrang entsprechend der DNA-Sequenz I wird durch Gelchromatographie gereinigt.

Das handelsübliche Plasmid pUC19 wird in bekannter Weise mit den Restriktionsendonukleasen SphI und EcoRI nach den Angaben der Hersteller geöffnet. Der Verdauungsansatz wird auf einem 1 %-igen Agarosegel durch Elektrophorese in bekannter Weise aufgetrennt und die Bruchstücke durch Anfärben mit Ethidiumbromid sichtbar gemacht. Die Plasmidbande wird anschließend aus dem Agarosegel herausgeschnitten und durch Elektroelution von der Agarose abgetrennt.

1 µg Plasmid wird dann mit 10 ng des DNA-Fragments entsprechend der DNA-Sequenz I über Nacht bei 16°C ligiert. Man erhält das Hybridplasmid gemäß Figur 1.

3. Konstruktion eines Expressionsplasmids, das die DNA-Sequenz I enthält.

Das Expressionsplasmid pWH1 besteht aus einem pBR322-Anteil und dem Gen für β-Galaktosidase mit dessen Regulationsregion. Die Herstellung von pWH1 wurde in der EP-A 0 133 282 ausführlich beschrieben. Dieses Plasmid enthält bei der Aminosäure 1005 der β-Galaktosidase eine singuläre EcoRI-Schnittstelle, die als Klonierungsstelle für eukaryotische Gene benutzt werden kann. Aus dem Plasmid gemäß Figur 1 kann die DNA-Sequenz I durch Restriktionsenzymverdauung mit SphI und EcoRI gewonnen werden. An die DNA-Sequenz I wird ein chemisch synthetischer Linker anligiert, der die Erkennungssequenzen für die Restriktionsenzyme SphI und EcoRI enthält:

```
5' AA TTC GGC GCA TG  3'
3'      G CCG C       5'
   (EcoRI)     (SphI)
```

Nach einer Restriktionsenzymverdauung mit EcoRI erhält man ein DNA-Fragment, flankiert von EcoRI Erkennungssequenzen. Ein solches Fragment kann anschließend in das mit EcoRI geöffnete Plasmid pWH1 integriert werden, wobei das Plasmid gemäß Figur 2 erhalten wird.

Hierzu wird 0,1-1 µg pWH1 mit EcoRI geöffnet und mit bakterieller alkalischer Phosphatase an den 5'-Enden der EcoRI-Schnittstelle dephosphoryliert. Hierdurch wird eine Ligation des Plasmids mit sich selber verhindert. Nur diejenigen Plasmide können ringförmig geschlossen werden, welche ein Genfragment mit flankierenden EcoRI-Erkennungssequenzen integriert haben. Hierzu werden 10 µg Calcitonin-Gen zugegeben und die Ligation durchgeführt.

4. Transformation der Hybridplasmide

Kompetente E. coli-Zellen werden mit 0,1 - 1 µg Plasmid gemäß Figur 2 transformiert und auf Ampicillin-Agarplatten plattiert. Anschließend läßt sich die Integration des Calcitonin-Gens und seine Integrationsrichtung im Plasmid durch DNA-Schnellaufarbeitung bestimmen (Maniatis, a.a.O.).

5. Expression

Nach Transformation dieser Hybridplasmide in E. coli wird ein Fusionsprotein exprimiert, das am Aminoende der Met-(Salm-Calcitonin-Gly)-Sequenz drei Aminosäuren aus dem Linker sowie die Sequenz der 1005 Aminosäuren der β-Galaktosidase trägt. Nach Bromcyan-Spaltung wird das Salm-Calcitonin-$Gly^{33}$ erhalten.

6. Aufarbeitung und Reinigung

Die zur gewünschten optischen Dichte kultivierten Bakterienstämme werden mit einem geeigneten Induktor, beispielsweise IPTG, hinreichend lange, beispielsweise 2 Stunden, induziert. Anschließend werden die Zellen mit 0,1 % Kresol und 0,1 mM Phenyl-methyl-sulfonylfluorid (Benzylsulfonylfluorid) abgetötet. Nach Zentrifugieren oder Filtrieren wird die Zellmasse in einer wäßrigsauren Lösung bei pH 3,0 in einer geeigneten Vorrichtung (French-Presse bzw. (R)Dyno-Mühle) aufgeschlossen, worauf die unlöslichen Bestandteile abzentrifugiert werden. Aus dem Überstand werden die Proteine nach Standard-Methoden (Grau, Müllner, Seipke, Angew. Chem. 98 (1986) 530) isoliert. Durch HPLC-Analytik werden die Anreicherung und die Reinheit der Produkte kontrolliert.

Das Methionin einschließlich des Anteils des am Methionin gebundenen Bakterienproteins β-Galaktosidase wird durch Bromcyan-Spaltung abgetrennt und das Calcitonin-Derivat extrahiert und gereinigt.

Fusionsproteine mit einem β-Galaktosidase-Anteil lassen sich bereits im Rohextrakt der lysierten Bakterien anhand ihres von der authentischen β-Galaktosidase unterschiedlichen Laufverhaltens in der Gelelektrophorese nachweisen. Nach der Bromcyan-Spaltung werden Reinheitsgrad und Anreicherung von Salm-Calcitonin-Gly über HPLC bestimmt.

Die Charakterisierung der Produkte auf Calcitonin-Aktivität kann immunologisch und biologisch erfolgen:

In einem Radioimmunoassay reagiert das bakteriologisch hergestellte Salm-Calcitonin I und seine Variante Salm-Calcitonin I-Gly mit einem Antikörper gegen synthetisches Salm-Calcitonin I.
Der Radioimmunoassay wird zweckmäßig als indirekter Immunpräzipitationsassay mit Anti-Calcitonin-Antikörper und Anti-IgC-Anti-Calcitonin-Antikörper ausgestaltet. Die Empfindlichkeit des RIA beträgt 10 ng Salm-Calcitonin I.

Die biologische Aktivität von Salm-Calcitonin I-$Gly^{33}$ bzw. von Salm-Calcitonin I kann durch Messung der Kalziumionenkonzentration im Serum bestimmt werden. Hierbei kann man auf kommerziell erhältliche Kalzium-"Assay Kits" zurückgreifen.

DNA-Sequenz I

| | | | | | | | |
|---|---|---|---|---|---|---|---|
| Triplett-Nr. | | | | 0 | 1 | 2 | 3 |
| Aminosäure | | | | Met | Cys | Ser | Asn |
| Nukleotid-Nr. | | 1 | 5 | | 10 | | 15 |
| Codierender Strang | 5' | | C | ATG | TGC | TCT | AAC |
| nicht codierender Strang | 3' | GT | ACG | TAC | ACG | AGA | TTG |

| | | | | | | | | | |
|---|---|---|---|---|---|---|---|---|---|
| 4 | 5 | 6 | 7 | 8 | 9 | 10 | 11 | 12 | 13 |
| Leu | Ser | Thr | Cys | Val | Leu | Gly | Lys | Leu | Ser |
| 20 | | 25 | | 30 | 35 | | 40 | | 45 |
| CTG | TCG | ACT | TGC | GTT | CTT | GGT | AAG | CTT | TCT |
| GAC | AGC | TGA | ACG | CAA | GAA | CCA | TTC | GAA | AGA |
| 14 | 15 | 16 | 17 | 18 | 19 | 20 | 21 | 22 | 23 |
| Gln | Glu | Leu | His | Lys | Leu | Gln | Thr | Tyr | Pro |
| 50 | | 55 | | 60 | 65 | | 70 | | 75 |
| CAG | GAA | CTT | CAT | AAA | CTG | CAG | ACC | TAT | CCG |
| GTC | CTT | GAA | GTA | TTT | GAC | GTC | TGG | ATA | GGC |
| 24 | 25 | 26 | 27 | 28 | 29 | 30 | 31 | 32 | 33 |
| Arg | Thr | Asn | Thr | Gly | Ser | Gly | Thr | Pro | Gly |
| 80 | | 85 | | 90 | 95 | | 100 | | 105 |
| CGC | ACT | AAT | ACC | GGC | TCT | GGT | ACC | CCT | GGT |
| GCG | TGA | TTA | TGG | CCG | AGA | CCA | TGG | GGA | CCA |

| | | | | |
|---|---|---|---|---|
| 34 | 35 | | | |
| Stp | Stp | | | |
| 110 | 115 | | | |
| TAA | TAG | . | | 3' |
| ATT | ATC | TTA | A | 5' |

## Ansprüche

1. Verfahren zur Herstellung von Salm-Calcitonin I mit zusätzlichem C-terminalen Glycin und von Salm-Calcitonin I-Carboxamid, wobei man in ein Expressionsplasmid ein Gen einbaut, das für das Glycyl-Peptid codiert, dieses exprimiert und gegebenenfalls das Glycyl-Peptid enzymatisch in das Carboxamid umwandelt, dadurch gekennzeichnet, daß das Strukturgen für das Glycyl-Peptid die DNA-Sequenz II

TGC TCT AAC CTG TCG ACT TGC GTT CTT GGT AAG
ACG AGA TTG GAC AGC TGA ACG CAA GAA CCA TCC
CTT TCT CAG GAA CTT CAT AAA CTG CAG ACC TAT
GAA AGA GTC CTT GAA GTA TTT GAC GTC TGG ATA

CCG CGC ACT AAT ACC GGC TCT GGT ACC CCT GGT
GGC GCG TGA TTA TGG CCG AGA CCA TGG GGA CCA
aufweist.

2. DNA-Sequenz II gemäß Anspruch 1.

3. DNA-Sequenz I (Anhang).

4. Verfahren zur Herstellung der DNA-Sequenz nach Anspruch 2 oder 3, dadurch gekennzeichnet, daß man den codierenden und den nicht-codierenden Strang nach dem Phosphitverfahren synthetisiert.

5. Hybridplasmid, dadurch gekennzeichnet, daß es die DNA-Sequenz I oder II enthält.

6. Hybridplasmid, das zwischen einer SphI-und einer EcoRI-Schnittstelle die DNA-Sequenz I enthält.

7. Wirtsorganismus, der ein Hybridplasmid nach Anspruch 5 oder 6 enthält.

8. E. coli, enthaltend ein Hybridplasmid nach Anspruch 5 oder 6.

Patentansprüche für die folgenden Vertragsstaaten: GR und AT

1. Verfahren zur Herstellung von Salm-Calcitonin I mit zusätzlichem C-terminalen Glycin und von Salm-Calcitonin I-Carboxamid, wobei man in ein Expressionsplasmid ein Gen einbaut, das für das Glycyl-Peptid codiert, dieses exprimiert und gegebenenfalls das Glycyl-Peptid enzymatisch in das Carboxamid umwandelt, dadurch gekennzeichnet, daß das Strukturgen für das Glycyl-Peptid die DNA-Sequenz II
TGC TCT AAC CTG TCG ACT TGC GTT CTT GGT AAG
ACG AGA TTG GAC AGC TGA ACG CAA GAA CCA TTC
CTT TCT CAG GAA CTT CAT AAA CTG CAG ACC TAT
GAA AGA GTC CTT GAA GTA TTT GAC GTC TGG ATA
CCG CGC ACT AAT ACC GGC TCT GGT ACC CCT GGT
GGC GCG TGA TTA TGG CCG AGA CCA TGG GGA CCA
aufweist.

2. Verfahren zur Herstellung des Strukturgens nach Anspruch 1, dadurch gekennzeichnet, daß man den codierenden und den nichtcodierenden Strang, gegebenenfalls mit flankierenden DNA-Abschnitten, chemisch synthetisiert.

3. Verfahren nach Anspruch 2, dadurch gekennzeichnet, daß die Synthese nach dem Phosphitverfahren erfolgt.

FIG.1

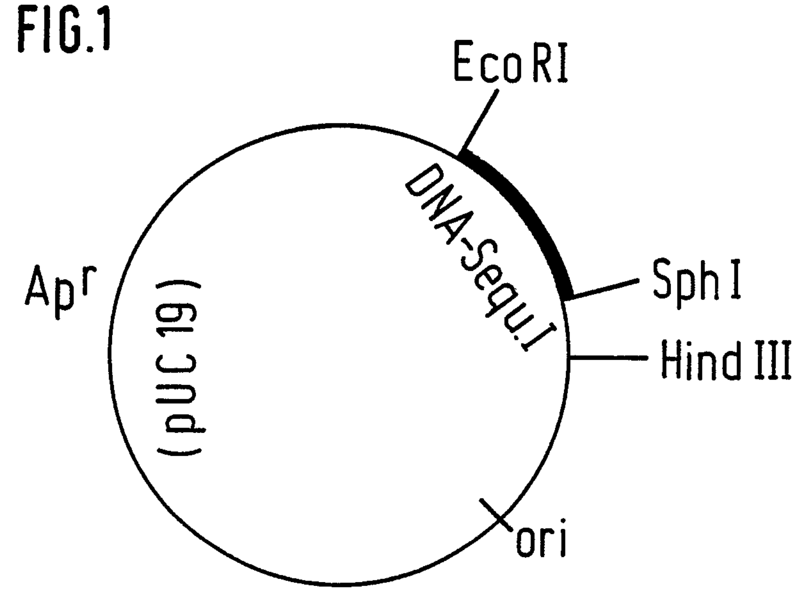
EcoRI
DNA-Sequ.I
SphI
HindIII
Apr
(pUC 19)
ori

FIG. 2

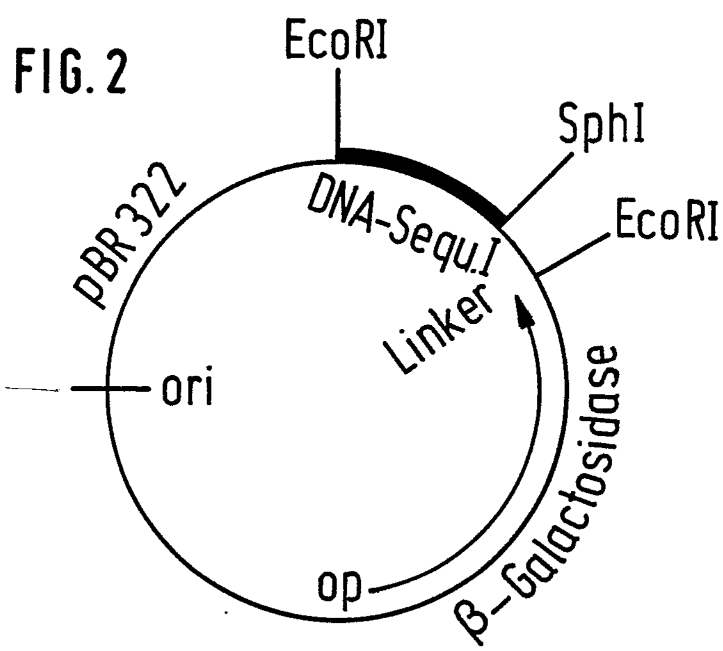
EcoRI
SphI
EcoRI
pBR322
DNA-Sequ.I
Linker
ori
op
β-Galactosidase

Europäisches Patentamt

# EUROPÄISCHER RECHERCHENBERICHT

Nummer der Anmeldung

EP 87 11 3445

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 191 869 (SAGAMI CHEMICAL RESEARCH CENTER)<br>* Seite 2, Zeile 36 - Seite 16, Zeile 2; Abbildungen 1,2,17; Ansprüche *<br>--- | 1 | C 12 N 15/00<br>C 07 H 21/04<br>C 07 K 7/36<br>C 12 N 1/20 //<br>C 12 P 21:02<br>C 12 R 1:19 |
| D,Y | EP-A-0 133 282 (HOECHST AG)<br>* Seite 1, Zeile 1 - Seite 3, Zeile 35; Ansprüche *<br>--- | 1 | |
| P,X | BIOLOGICAL ABSTRACTS,RRM, Band 33, 1987, Nr. 84491, Philadelphia, US; F. HEIN et al.: "Use of long oligonucleotides in gene synthesis chemical synthesis and cloning of a gene for salmon calcitonin" & Nucleosides nucleotides, Band 6, Nr. 1-2, Seite 489-490<br>* Titel *<br>----- | 1-8 | |
| | | | **RECHERCHIERTE SACHGEBIETE (Int. Cl.4)** |
| | | | C 12 N |

Der vorliegende Recherchenbericht wurde für alle Patentansprüche erstellt

| Recherchenort | Abschlußdatum der Recherche | Prüfer |
|---|---|---|
| DEN HAAG | 02-12-1987 | YEATS S.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)